# EUROPEAN PATENT APPLICATION

(11) **EP 4 714 445 A1**
(43) Date of publication of application: **25.03.2026**
(21) Application number: 24832195.2
(22) Date of filing: 17.04.2024
(51) Int. Cl.: A61K 31/42, A61K 31/194, A61K 31/341, A61K 31/519, A61P 27/02, A23L 33/10

(54) **NOVEL COMPOSITION FOR PREVENTING OR TREATING RETINAL DISEASES**

(30) Priority: 27.06.2023 KR 20230082567
(71) Applicant: Seoul National University Hospital, Seoul 03080 (KR); Seoul National University R&DB Foundation, Seoul 08826 (KR)
(72) Inventor: PARK, Kyu Hyung, Seongnam-si, Gyeonggi-do 13597 (KR); JOO, Kwangsic, Seongnam-si, Gyeonggi-do 13620 (KR); CHOI, Seung Woo, Seongnam-si, Gyeonggi-do 13620 (KR); JEON, Gyeong Yun, Seongnam-si, Gyeonggi-do 13620 (KR); KIM, Minji, Seongnam-si, Gyeonggi-do 13620 (KR)
(74) Representative: Dentons Patent Solutions Rechtsanwaltsgesellschaft mbH
(86) International application number: PCT/KR2024/005151
(87) International publication number: WO 2025/005410

(57) **Abstract**

The present specification discloses a novel composition for preventing or treating retinal diseases. In one aspect, compounds in the present invention are extremely effective in protecting cells against the cytotoxicity of all-trans-retinal, inhibiting cellular aging, and inhibiting intracellular reactive oxygen species, and thus can be used in therapeutic agents for retinal diseases such as hereditary macular degeneration and age-related macular degeneration.

## Description

### [Technical Field]

### [CROSS-REFERENCE TO RELATED APPLICATION]

This application claims priority to and the benefit of Korean Patent Application No. 10-2023-0082567, filed on June 27, 2023, entitled "Novel composition for preventing or treating retinal diseases," the disclosure of which is incorporated herein by reference in its entirety.

This specification describes a novel composition for preventing or treating retinal diseases.

Meanwhile, the present application was supported by the following national research and development project.

### [National research and development project that has supported the present invention]

[Project Number] 1711166085
[Grant Number] 2020R1A2C2011189
[Implementing Agency] Ministry of Science and ICT
[Research Management Agency] National Research Foundation of Korea
[Project Name] Individual Basic Research (Ministry of Science and ICT)
[Project Title] Drug Repurposing Focusing on Genetic Pathways in Hereditary Retinal Diseases
[Contribution Rate] 1/2
[Organization] Seoul National University
[Research Period] March 1, 2020 to February 28, 2023

### [National research and development project that has supported the present invention]

[Project Number] 1711147389
[Grant Number] 2019R1C1C1009345
[Implementing Agency] Ministry of Science and ICT
[Research Management Agency] National Research Foundation of Korea
[Project Name] Individual Basic Research (Ministry of Science and ICT) (R&D)
[Project Title] Identification of Pathological Mechanisms of Retinal Diseases through Correlation Analysis of Blood Microbiomes and Human Genomes
[Contribution Rate] 1/2
[Organizer] Seoul National University Bundang Hospital
[Research Period] March 1, 2019 to February 28, 2022

### [Background Art]

ABCA4 is present in the photoreceptor cells of the retina and serves as a conduit protein that receives external light stimulation and transports all-trans-retinal, which is essential for the visual cycle, from the photoreceptor cells to the retinal pigment epithelium cells. Diseases caused by genetic mutations in ABCA4, one of the three most common causative genes of hereditary retinal diseases, result in premature blindness, especially at a young age. Diseases caused by ABCA4 genetic mutations include Stargardt disease, cone dystrophy, and cone-rod dystrophy. Due to genetic mutations in ABCA4, all-trans-retinal accumulates in retinal epithelial cells, and this accumulates as a photooxidant in the form of an all-trans-retinal dimer, A2E, through an oxidative mechanism. This toxic substance is not easily degraded and is difficult to excrete, causing a toxic reaction in retinal photoreceptor cells, leading to cell death.

Age-related macular degeneration is the most common cause of blindness in people over 60 worldwide and is a representative disease caused by aging. Age-related macular degeneration may be classified into early dry, late wet, and late atrophic forms. However, there is currently no therapeutic agent for treating the early dry form or for preventing blindness from progressing to the late atrophic form in advanced macular degeneration. Macular degeneration is well known to share similarities with ABCA4-associated retinal diseases in terms of genetics and pathogenesis, and the main component of drusen, the causative deposit of macular degeneration, is A2E.

Meanwhile, only one therapeutic agent for hereditary retinal diseases is currently known, which is a drug targeting the RPE65 gene. However, only a very small number of patients are eligible for this therapeutic agent, and for most hereditary retinal diseases, no treatment is available, leaving patients to simply watch as they progress to blindness.

Accordingly, the present inventors have made efforts to discover a drug that removes or reduces the cytotoxicity and photooxidation of all-trans-retinal, and as a result, they have confirmed that the compounds of the present invention not only have an excellent cytoprotective effect against cytotoxicity caused by all-trans-retinal and an inhibitory effect against cell aging caused by hydrogen peroxide, but can also effectively suppress the increase in intracellular reactive oxygen species caused by all-trans-retinal and reduce the intracellular reactive oxygen species level to almost below the level of normal cells to which all-trans-retinal is not added, thereby completing the present invention.

### [Disclosure]

### [Technical Problem]

An object of the present invention is, in one aspect, to provide a novel composition for preventing, ameliorating, or treating retinal diseases.

### [Technical Solution]

In order to achieve the above-described object, the present invention provides a composition for preventing, ameliorating, or treating retinal diseases, which includes as an active ingredient one or more compounds represented by the following General Formulas 1 to 5.

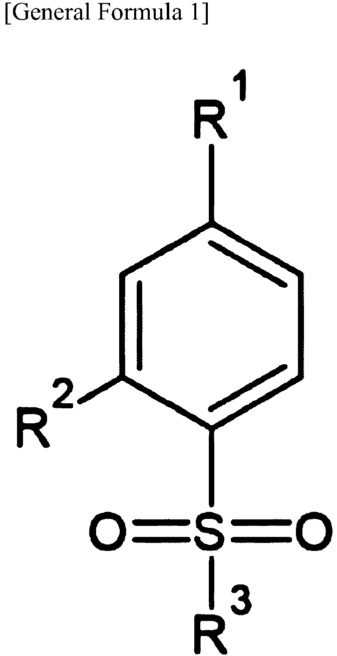

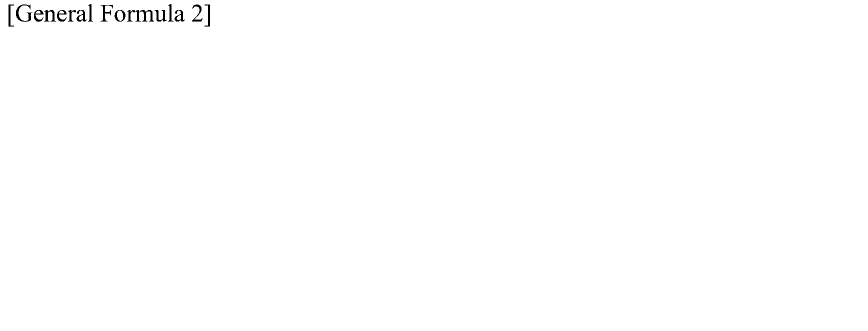

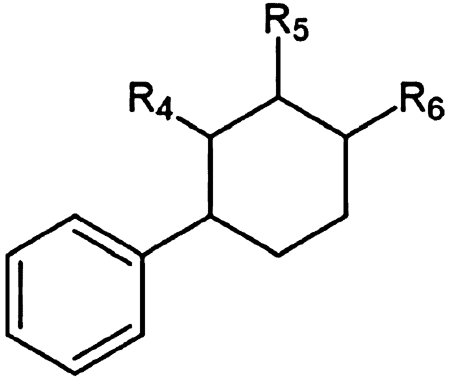

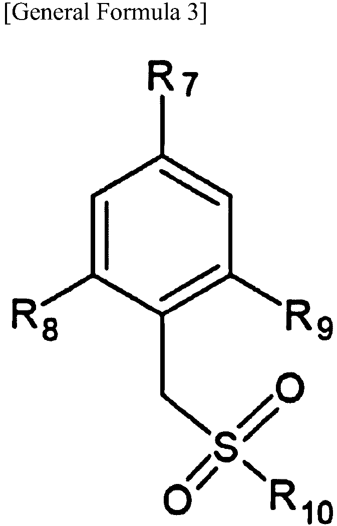

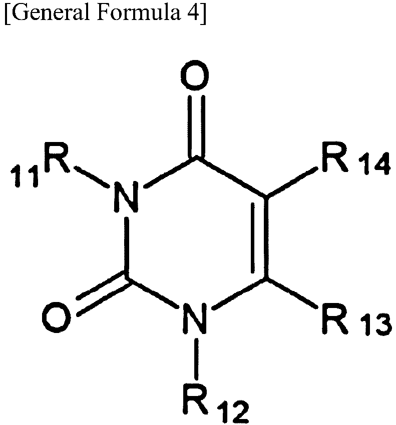

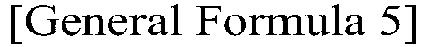

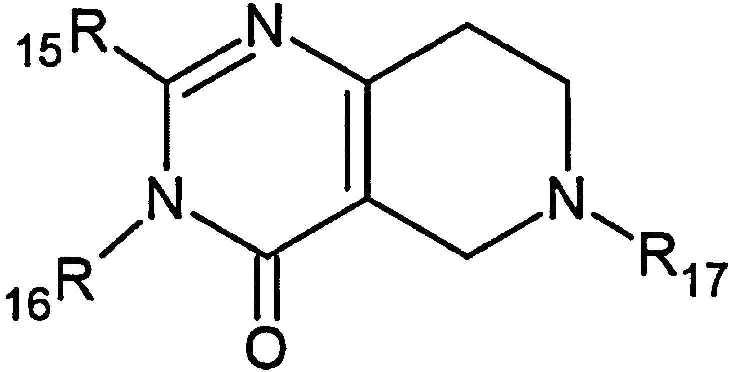

In one exemplary embodiment, R1 to R17 of General Formulas 1 to 5 may each be a substituent selected from the group consisting of a hydrogen, a deuterium, a halogen atom, a hydroxyl group, a cyano group, an amino group, an amido group, a nitro group, a substituted or unsubstituted (C1-C30) alkyl group, a substituted or unsubstituted (C3-C30) cycloalkyl group, a substituted or unsubstituted (C1-C30) heteroalkyl group, a substituted or unsubstituted (C2-C30) heterocycloalkyl group, a substituted or unsubstituted (C6-C30 )aryl (C1-C30) alkyl group, an alkoxy group, an aryloxy group, a silyl group, a substituted or unsubstituted (C2-C30) alkenyl group, a substituted or unsubstituted (C2-C30) cycloalkenyl group, a substituted or unsubstituted (C1-C30) heteroalkenyl group, a substituted or unsubstituted (C2-C30) alkynyl group, a substituted or unsubstituted (C6-C30) aryl group, a substituted or unsubstituted (C2-C30) heteroaryl group, an acyl group, a carboxylic acid, an ether, an ester, a nitrile, an isonitrile, a sulfanyl group, a sulfinyl group, a sulfonyl group, a phosphino group, and combinations thereof.

In one exemplary embodiment, R13 and R14 may be present independently or may be fused to form a ring.

In one exemplary embodiment, the retinal disease may be one or more selected from the group consisting of hereditary macular degeneration, age-related macular degeneration, myopic macular degeneration, and macular degeneration caused by inflammation.

In one exemplary embodiment, the hereditary macular degeneration may be one or more selected from the group consisting of retinitis pigmentosa, vitelliform macular dystrophy, Stargardt disease, X-linked retinoschisis, and cone dystrophy.

In one exemplary embodiment, the macular degeneration caused by inflammation may be macular degeneration caused by Behcet's uveitis.

In one exemplary embodiment, the compound may have one or more effects of cell death inhibition, cell aging inhibition, or intracellular reactive oxygen species inhibition.

In one exemplary embodiment, the composition may be one or more of a pharmaceutical composition, a quasi-drug composition, and a health functional food composition.

### [Advantageous Effects]

In one aspect, the compounds of the present invention have excellent cytoprotective effects against cytotoxicity caused by all-trans-retinal, inhibitory effects against cell aging, and inhibitory effects against intracellular reactive oxygen species, and thus can be used as drugs that can treat and inhibit the progression of retinal diseases such as hereditary macular degeneration and age-related macular degeneration.

### [Description of Drawings]

FIGS. 1A and 1B show the results of treating RPE-1, a retinal pigment epithelial cell line, with various concentrations of all-trans-retinal (ATRL).
FIG. 2 shows the selection of compounds that satisfy lead likeness, the Lipinski rule, and the Ghose filter.
FIGS. 3A to 3C show the real-time cytoprotective effects of the final candidate compounds against all-trans-retinal cytotoxicity.
FIG. 4 shows the cytotoxicity of the final candidate compounds themselves.
FIGS. 5 and 6 show the inhibitory effects against cellular senescence of the final candidate compounds.
FIG. 7 shows the reactive oxygen species inhibition activity of compound #17, one of the final candidate compounds.

### [Modes of the Invention]

The present invention is described in detail.

The present invention, in one aspect, relates to a composition for preventing, ameliorating, or treating retinal diseases, including as an active ingredient one or more compounds represented by the following General Formulas 1 to 5:

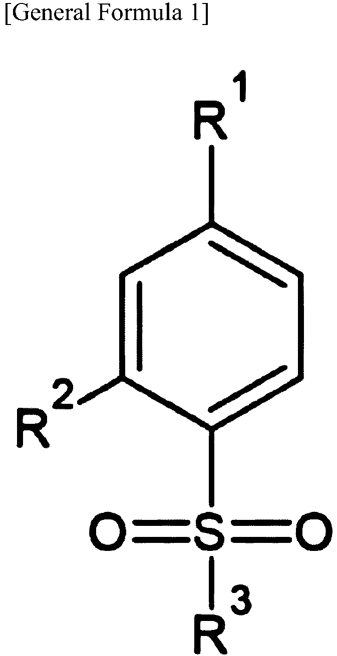

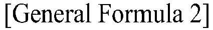

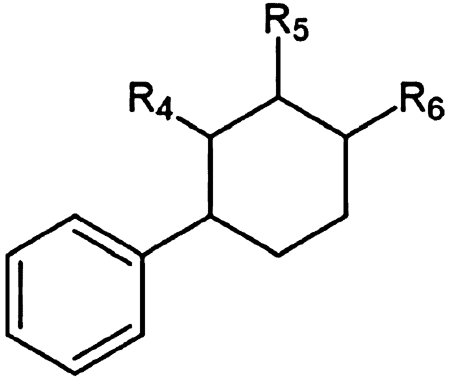

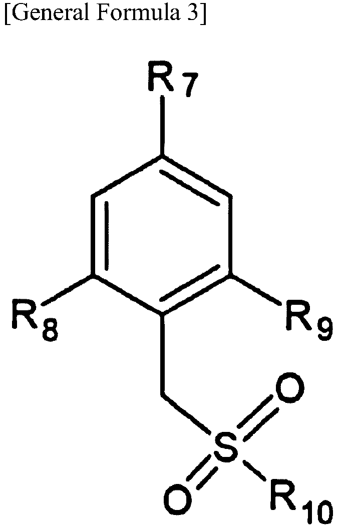

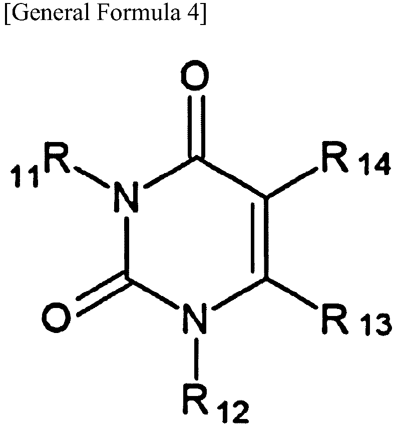

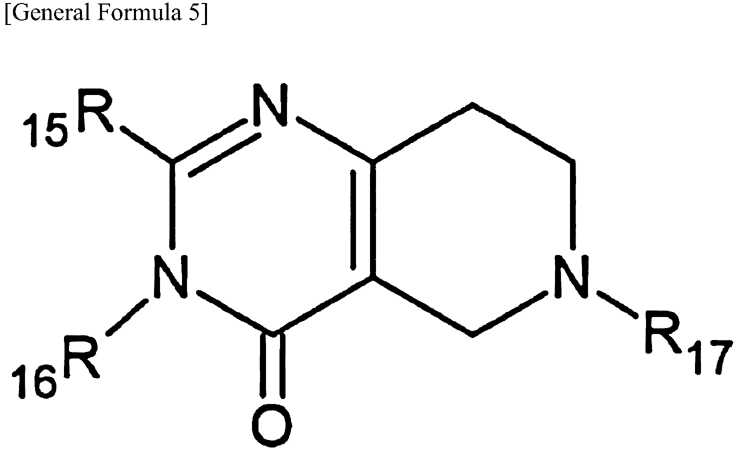

In one exemplary embodiment, R1 to R3 of General Formula 1 may each be a substituent selected from the group consisting of a hydrogen, a deuterium, a halogen atom, a hydroxyl group, a cyano group, an amino group, an amido group, a nitro group, a substituted or unsubstituted (C1-C30) alkyl group, a substituted or unsubstituted (C3-C30) cycloalkyl group, a substituted or unsubstituted (C1-C30) heteroalkyl group, a substituted or unsubstituted (C2-C30) heterocycloalkyl group, a substituted or unsubstituted (C6-C30) aryl (C1-C30) alkyl group, an alkoxy group, an aryloxy group, a silyl group, a substituted or unsubstituted (C2-C30) alkenyl group, a substituted or unsubstituted (C2-C30) cycloalkenyl group, a substituted or unsubstituted (C1-C30) heteroalkenyl group, a substituted or unsubstituted (C2-C30) alkynyl group, a substituted or unsubstituted (C6-C30) aryl group, a substituted or unsubstituted (C2-C30) heteroaryl group, an acyl group, a carboxylic acid, an ether, an ester, a nitrile, an isonitrile, a sulfanyl group, a sulfinyl group, a sulfonyl group, a phosphino group, and combinations thereof.

In one exemplary embodiment, R1 may be a substituent selected from the group consisting of a hydrogen, a deuterium, a halogen atom, and combinations thereof, R2 may be a substituent selected from the group consisting of a hydroxyl group, a cyano group, an amino group, an amido group, a nitro group, an alkoxy group, an aryloxy group, a silyl group, an acyl group, a carboxylic acid group, an ether group, an ester group, a nitrile, an isonitrile, a sulfanyl group, a sulfinyl group, a sulfonyl group, a phosphino group, and combinations thereof, and R3 may be a substituent selected from the group consisting of a substituted or unsubstituted (C1-C30) heteroalkyl group, a substituted or unsubstituted (C2-C30) heterocycloalkyl group, a substituted or unsubstituted (C1-C30) heteroalkenyl group, a substituted or unsubstituted (C2-C30) heteroaryl group, and combinations thereof.

In one exemplary embodiment, R1 may be a halogen atom, R2 may be a cyano group, and R3 may be a substituted or unsubstituted (C2-C30) heterocycloalkyl group. For example, General Formula 1 may be Chemical Formula 1, but is not limited thereto:

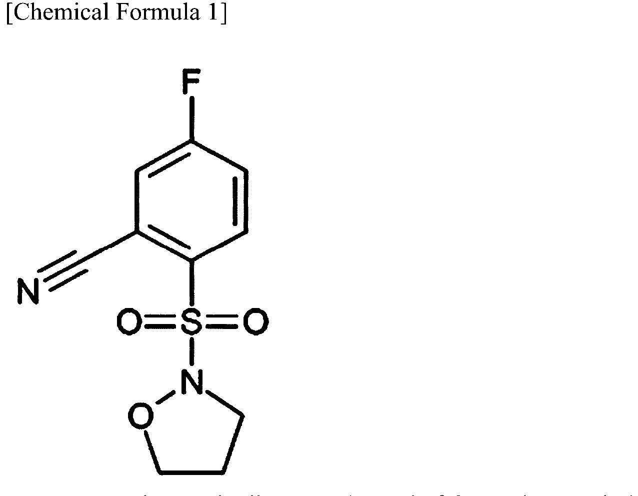

In one exemplary embodiment, R4 to R6 of General Formula 2 may each be a substituent selected from the group consisting of a hydrogen, a deuterium, a halogen atom, a hydroxyl group, a cyano group, an amino group, an amido group, a nitro group, a substituted or unsubstituted (C1-C30) alkyl group, a substituted or unsubstituted (C3-C30) cycloalkyl group, a substituted or unsubstituted (C1-C30) heteroalkyl group, a substituted or unsubstituted (C2-C30) heterocycloalkyl group, a substituted or unsubstituted (C6-C30) aryl (C1-C30) alkyl group, an alkoxy group, an aryloxy group, a silyl group, a substituted or unsubstituted (C2-C30) alkenyl group, a substituted or unsubstituted (C2-C30) cycloalkenyl group, a substituted or unsubstituted (C1-C30) heteroalkenyl group, a substituted or unsubstituted (C2-C30) alkynyl group, a substituted or unsubstituted (C6-C30) aryl group, a substituted or unsubstituted (C2-C30) heteroaryl group, an acyl group, a carboxylic acid, an ether, an ester, a nitrile, an isonitrile, a sulfanyl group, a sulfinyl group, a sulfonyl group, a phosphino group, and combinations thereof.

In one exemplary embodiment, R4 may be a substituent selected from the group consisting of a hydrogen, a deuterium, a substituted or unsubstituted (C1-C30) alkyl group, and combinations thereof, and R5 or R6 may each be a substituent selected from the group consisting of a hydroxyl group, a cyano group, an amino group, an amido group, a nitro group, an alkoxy group, an aryloxy group, a silyl group, an acyl group, a carboxylic acid, an ether, an ester, a nitrile, an isonitrile, a sulfanyl group, a sulfinyl group, a sulfonyl group, a phosphino group, and combinations thereof.

In one exemplary embodiment, R4 may be a substituted or unsubstituted (C1-C5) alkyl group, and R5 or R6 may each be a carboxylic acid or an ester. For example, General Formula 2 may be Chemical Formula 2, but is not limited thereto:

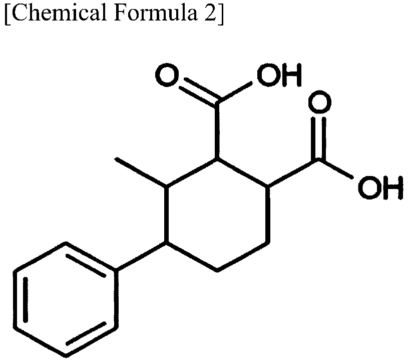

In one exemplary embodiment, R7 to R10 of General Formula 3 may each be a substituent selected from the group consisting of a hydrogen, a deuterium, a halogen atom, a hydroxyl group, a cyano group, an amino group, an amido group, a nitro group, a substituted or unsubstituted (C1-C30) alkyl group, a substituted or unsubstituted (C3-C30) cycloalkyl group, a substituted or unsubstituted (C1-C30) heteroalkyl group, a substituted or unsubstituted (C2-C30) heterocycloalkyl group, a substituted or unsubstituted (C6-C30) aryl (C1-C30) alkyl group, an alkoxy group, an aryloxy group, a silyl group, a substituted or unsubstituted (C2-C30) alkenyl group, a substituted or unsubstituted (C2-C30) cycloalkenyl group, a substituted or unsubstituted (C1-C30) heteroalkenyl group, a substituted or unsubstituted (C2-C30) alkynyl group, a substituted or unsubstituted (C6-C30) aryl group, a substituted or unsubstituted (C2-C30) heteroaryl group, an acyl group, a carboxylic acid, an ether, an ester, a nitrile, an isonitrile, a sulfanyl group, a sulfinyl group, a sulfonyl group, a phosphino group, and combinations thereof.

In one exemplary embodiment, R7 to R9 may each be a substituent selected from the group consisting of a hydrogen, a deuterium, a substituted or unsubstituted (C1-C30) alkyl group, and combinations thereof, and R10 may be a substituent selected from the group consisting of a substituted or unsubstituted (C1-C30) alkyl group, a substituted or unsubstituted (C3-C30) cycloalkyl group, a substituted or unsubstituted (C1-C30) heteroalkyl group, a substituted or unsubstituted (C2-C30) heterocycloalkyl group, a substituted or unsubstituted (C6-C30) aryl (C1-C30) alkyl group, a substituted or unsubstituted (C1-C30) heteroalkenyl group, a substituted or unsubstituted (C2-C30) heteroaryl group, and combinations thereof.

In one exemplary embodiment, R7 to R9 may each be a substituted or unsubstituted (C1-C30) alkyl group, and R10 may be a substituent selected from the group consisting of a substituted or unsubstituted (C1-C30) alkyl group, a substituted or unsubstituted (C3-C30) cycloalkyl group, a substituted or unsubstituted (C1-C30) heteroalkyl group, a substituted or unsubstituted (C2-C30) heterocycloalkyl group, and combinations thereof. For example, General Formula 3 may be Chemical Formula 3, but is not limited thereto:

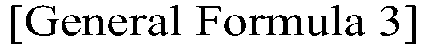

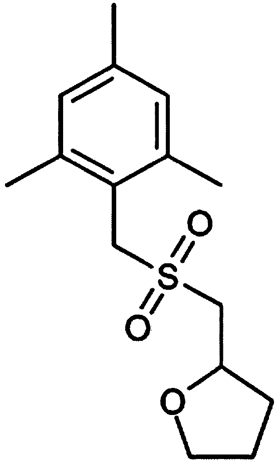

In one exemplary embodiment, R11 to R14 of General Formula 4 may each be a substituent selected from the group consisting of a hydrogen, a deuterium, a halogen atom, a hydroxyl group, a cyano group, an amino group, an amido group, a nitro group, a substituted or unsubstituted (C1-C30) alkyl group, a substituted or unsubstituted (C3-C30) cycloalkyl group, a substituted or unsubstituted (C1-C30) heteroalkyl group, a substituted or unsubstituted (C2-C30) heterocycloalkyl group, a substituted or unsubstituted (C6-C30) aryl (C1-C30) alkyl group, an alkoxy group, an aryloxy group, a silyl group, a substituted or unsubstituted (C2-C30) alkenyl group, a substituted or unsubstituted (C2-C30) cycloalkenyl group, a substituted or unsubstituted (C1-C30) heteroalkenyl group, a substituted or unsubstituted (C2-C30) alkynyl group, a substituted or unsubstituted (C6-C30) aryl group, a substituted or unsubstituted (C2-C30) heteroaryl group, an acyl group, a carboxylic acid, an ether, an ester, a nitrile, an isonitrile, a sulfanyl group, a sulfinyl group, a sulfonyl group, a phosphino group, and combinations thereof, and R13 and R14 may be present independently or may be fused to form a ring.

In one exemplary embodiment, R11 may be a substituent selected from the group consisting of a substituted or unsubstituted (C1-C30) alkyl group, a substituted or unsubstituted (C3-C30) cycloalkyl group, a substituted or unsubstituted (C1-C30) heteroalkyl group, a substituted or unsubstituted (C2-C30) heterocycloalkyl group, a substituted or unsubstituted (C6-C30) aryl (C1-C30) alkyl group, a substituted or unsubstituted (C2-C30) alkenyl group, a substituted or unsubstituted (C2-C30) cycloalkenyl group, a substituted or unsubstituted (C1-C30) heteroalkenyl group, a substituted or unsubstituted (C2-C30) alkynyl group, a substituted or unsubstituted (C6-C30) aryl group, a substituted or unsubstituted (C2-C30) heteroaryl group, and combinations thereof, R12 may be a substituent selected from the group consisting of a hydrogen, a deuterium, a substituted or unsubstituted (C1-C30) alkyl group, and combinations thereof, and R13 and R14 may be fused to form a substituted or unsubstituted ring.

In one exemplary embodiment, R11 may be a substituent selected from the group consisting of a substituted or unsubstituted (C2-C30) cycloalkenyl group, a substituted or unsubstituted (C3-C30) cycloalkyl group, a substituted or unsubstituted (C2-C30) heterocycloalkyl group, and combinations thereof, R12 may be a hydrogen or a deuterium, and R13 and R14 may be fused to form a substituted or unsubstituted (C2-C30) heterocycle. For example, General Formula 4 may be Chemical Formula 4, but is not limited thereto.

In one exemplary embodiment, R11 may be a substituted or unsubstituted (C6-C30) aromatic ring.

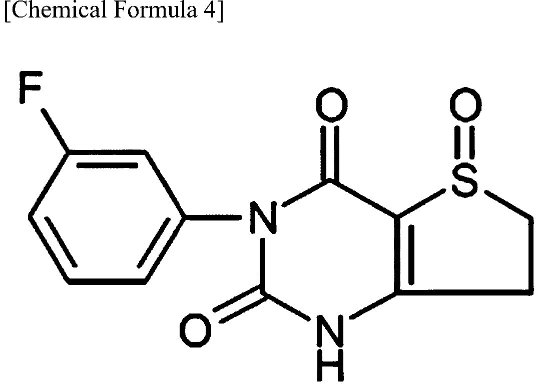

In one exemplary embodiment, R15 to R17 of General Formula 5 may each be a substituent selected from the group consisting of a hydrogen, a deuterium, a halogen atom, a hydroxyl group, a cyano group, an amino group, an amido group, a nitro group, a substituted or unsubstituted (C1-C30) alkyl group, a substituted or unsubstituted (C3-C30) cycloalkyl group, a substituted or unsubstituted (C1-C30) heteroalkyl group, a substituted or unsubstituted (C2-C30) heterocycloalkyl group, a substituted or unsubstituted (C6-C30) aryl (C1-C30) alkyl group, an alkoxy group, an aryloxy group, a silyl group, a substituted or unsubstituted (C2-C30) alkenyl group, a substituted or unsubstituted (C2-C30) cycloalkenyl group, a substituted or unsubstituted (C1-C30) heteroalkenyl group, a substituted or unsubstituted (C2-C30) alkynyl group, a substituted or unsubstituted (C6-C30) aryl group, a substituted or unsubstituted (C2-C30) heteroaryl group, an acyl group, a carboxylic acid, an ether, an ester, a nitrile, an isonitrile, a sulfanyl group, a sulfinyl group, a sulfonyl group, a phosphino group, and combinations thereof.

In one exemplary embodiment, R15 may be a substituent selected from the group consisting of a substituted or unsubstituted (C1-C30) alkyl group, a substituted or unsubstituted (C3-C30) cycloalkyl group, a substituted or unsubstituted (C1-C30) heteroalkyl group, a substituted or unsubstituted (C2-C30) heterocycloalkyl group, a substituted or unsubstituted (C6-C30) aryl (C1-C30) alkyl group, a substituted or unsubstituted (C1-C30) heteroalkenyl group, a substituted or unsubstituted (C2-C30) heteroaryl group, and combinations thereof, R16 may be a substituent selected from the group consisting of a hydrogen, a deuterium, a substituted or unsubstituted (C1-C30) alkyl group, and combinations thereof, and R17 may be a substituent selected from the group consisting of a substituted or unsubstituted (C1-C30) alkyl group, a substituted or unsubstituted (C3-C30) cycloalkyl group, a (C1-C30) heteroalkyl group, a substituted or unsubstituted (C2-C30) heterocycloalkyl group, an amino group, an amido group, a nitro group, a carboxylic acid, an ester, and combinations thereof.

In one exemplary embodiment, R15 may be a substituted or unsubstituted (C2-C30) heterocycloalkyl group, R16 may be a hydrogen or a deuterium, and R17 may be a substituent selected from the group consisting of a substituted or unsubstituted (C1-C30) alkyl group, a substituted or unsubstituted (C3-C30) cycloalkyl group, an amino group, an amido group, a nitro group, a carboxylic acid, an ester, and combinations thereof. For example, General Formula 5 may be Chemical Formula 5, but is not limited thereto:

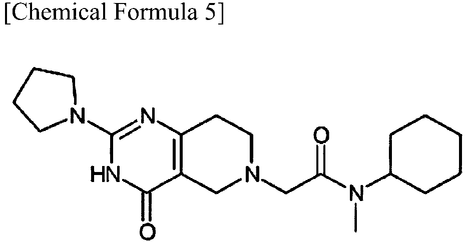

In one exemplary embodiment, the compound represented by [Chemical Formula 1] may be named 5-fluoro-2-(isoxazolidin-2-ylsulfonyl)benzonitrile.

In one exemplary embodiment, the compound represented by [Chemical Formula 2] may be named 3-methyl-4-phenylcyclohexane-1,2-dicarboxylic acid.

In one exemplary embodiment, the compound represented by [Chemical Formula 3] may be named 2-(((2,4,6-trimethylbenzyl)sulfonyl)methyl)tetrahydrofuran.

In one exemplary embodiment, the compound represented by [Chemical Formula 4] may be named 3-(3-fluorophenyl)-6,7-dihydrothieno[3,2-d]pyrimidine-2,4(1H,3H)-dione 5-oxide.

In one exemplary embodiment, the compound represented by [Chemical Formula 5] may be named N-cyclohexyl-N-methyl-2-(4-oxo-2-(pyrrolidin-1-yl)-3,5,7,8-tetrahydropyrido[4,3-d]pyrimidin-6(4H)-yl)acetamide.

In one exemplary embodiment, the composition may include the compound at a concentration of 0.1 µM or more, 10 µM or more, 25 µM or more, 50 µM or more, or 100 µM or more, or 1000 µM or less, 750 µM or less, 500 µM or less, 450 µM or less, 400 µM or less, 350 µM or less, or 300 µM or less. For example, the composition may include the compound at a concentration of 0.1 µM to 1000 µM or 10 µM to 500 µM, and when the compound is included at a concentration of less than 0.1 µM, the composition may have poor cell death inhibition, cell senescence inhibition, or intracellular reactive oxygen species inhibition, or when the compound is included at a concentration exceeding 1000 µM, it may exhibit high cytotoxicity.

In one exemplary embodiment, the retinal disease may be one or more selected from the group consisting of hereditary macular degeneration, age-related macular degeneration, myopic macular degeneration, and macular degeneration caused by inflammation, but is not limited thereto.

In one exemplary embodiment, the hereditary macular degeneration may be one or more selected from the group consisting of retinitis pigmentosa, vitelliform macular dystrophy, Stargardt disease, X-linked retinoschisis, and cone dystrophy.

In one exemplary embodiment, the macular degeneration caused by inflammation may be macular degeneration caused by Behcet's uveitis

In one exemplary embodiment, the compound may inhibit cell death induced by all-trans-retinal, thereby exhibiting cytoprotective effects against cytotoxicity induced by all-trans-retinal. The compound includes compounds of General Formulas 1 to 5 as well as derivatives and analogs thereof.

In one exemplary embodiment, the compound may exhibit inhibitory activity against cellular aging induced by hydrogen peroxide. The compound may include compounds of General Formulas 1 to 5, including derivatives and analogs thereof.

In one exemplary embodiment, the compound may reduce intracellular reactive oxygen species levels due to all-trans-retinal. The compound may include compounds of General Formulas 1 to 5, including derivatives and analogs thereof.

In one exemplary embodiment, the composition may be one or more of a pharmaceutical composition, a quasi-drug composition, and a health functional food composition.

In one exemplary embodiment, the pharmaceutical composition may be formulated into a unit dosage form suitable for administration to a patient's body according to a conventional method and administered. Suitable dosage forms for this purpose include parenterally administered preparations such as injections or topical preparations. Injectable dosage forms are preferably isotonic aqueous solutions or suspensions. However, diluents or excipients such as commonly used fillers, bulking agents, binders, wetting agents, disintegrants, and surfactants may also be used. The preparation of the present invention thus prepared may be administered parenterally, for example, directly to a specific site, according to a conventional method. In this case, the composition may be injected using a syringe. It should be understood that the actual dosage of the active ingredient should be determined in light of various relevant factors, such as the administration route and the patient's weight, age, and sex.

In one exemplary embodiment, the quasi-drug composition may be added as is or used together with other quasi-drugs or quasi-drug ingredients, and may be used appropriately according to a conventional method. The mixing amount of the active ingredient may be appropriately determined depending on the intended use. The ingredients included in the quasi-drug composition may include, in addition to the active ingredient, ingredients commonly used in quasi-drug compositions, and may include, for example, an abrasive, a wetting agent, a binder, a foaming agent, a sweetener, a preservative, a medicinal ingredient, a flavoring agent, a coloring agent, a solvent, a whitening agent, a solubilizer, or a pH adjuster. The quasi-drug composition may be prepared in any formulation commonly prepared in the art.

In one exemplary embodiment, the health functional food composition may include a food additive acceptable from a food science perspective, and may further include suitable carriers, excipients, and diluents commonly used in the manufacture of foods. In addition to the above, the composition may further include various nutrients, vitamins, electrolytes, flavoring agents, coloring agents, pectic acid and salts thereof, alginic acid and salts thereof, organic acids, protective colloid thickeners, pH adjustors, stabilizers, preservatives, glycerin, alcohol, carbonating agents used in carbonated beverages, and the like.

In another aspect, the present invention relates to a method for preventing, ameliorating, or treating retinal diseases by administering an effective amount of one or more of the compounds represented by General formulas 1 to 5 to a subject.

In another aspect, the present invention relates to the use of one or more of the compounds represented by General formulas 1 to 5 for the preparation of a composition for preventing, ameliorating, or treating retinal diseases.

In another aspect, the present invention relates to the use of any one or more of the compounds represented by General formulas 1 to 5 for the preparation of a pharmaceutical composition for preventing or treating retinal diseases.

In another aspect, the present invention relates to the use of any one or more of the compounds represented by General formulas 1 to 5 for the preparation of a quasi-drug composition for preventing, ameliorating, or treating retinal diseases.

In another aspect, the present invention relates to the use of any one or more of the compounds represented by General formulas 1 to 5 for the preparation of a health food composition for preventing or ameliorating retinal diseases.

In another aspect, the present invention relates to the use of any one or more of the compounds represented by General formulas 1 to 5 for preventing, ameliorating, or treating retinal diseases.

In another aspect, the present invention relates to the non-therapeutic or therapeutic use of any one or more of the compounds represented by General formulas 1 to 5.

Hereinafter, the present invention will be described in more detail through examples and the like. These examples are intended solely to illustrate the present invention, and it will be apparent to those skilled in the art that the scope of the present invention is not to be construed as being limited by these examples.

### [Examples]

### [Example 1] Compound screening

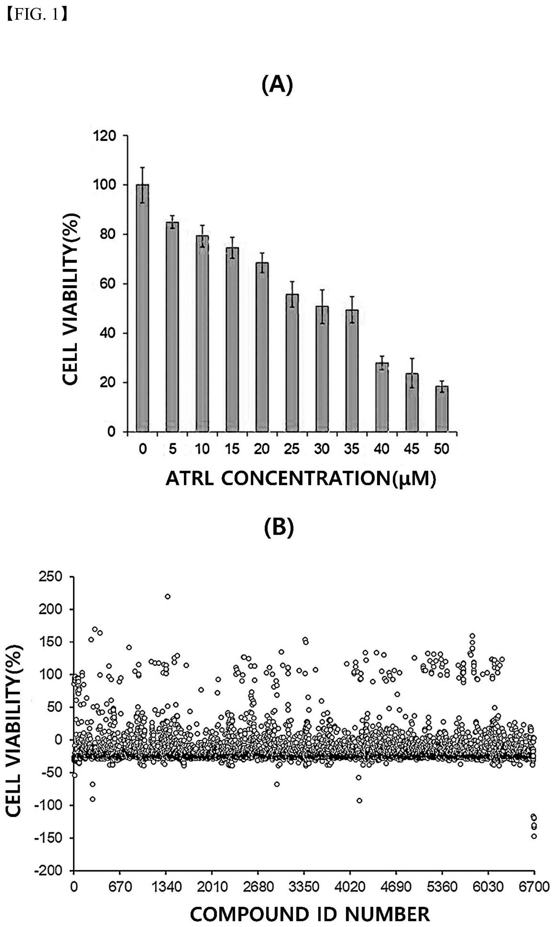

As a result of treating the RPE-1 cell line, a retinal pigment epithelial cell line, with various concentrations of all-trans-retinal (ATRL), a significant decrease was observed in RPE-1 cell viability as the concentration of all-trans-retinal increased (FIGS. 1A and 1B).

Based on the above-described results, a viability test was performed on 6,696 compounds using all-trans-retinal at a concentration of 30 µM. The highest cell viability was 219%, and the top 197 compounds that significantly increased cell viability compared to the control were selected. The top six compounds are listed in Table 1, and the 1,364th compound showed a 219% increase in cell viability compared to the control (Table 1).

**[Table 1]**

| **Cell viability of the top six compounds** | | | | | |
|---|---|---|---|---|---|
| **No** | **Position** | **Concentration (mM)** | **Volume (µl)** | **Tested concentration (mM)** | **% viability** |
| **1364** | **RPL-D1-000191-D03** | **5.2** | **5** | **0.25** | **219.159082** |
| **306** | **RPL-D1-000177-B11** | **5.1** | **5** | **0.24** | **169.055534** |
| **384** | **RPL-D1-000178-H10** | **5.2** | **5** | **0.25** | **163.741893** |
| **5795** | **RPL-D1-000246-C07** | **5.4** | **5** | **0.26** | **159.466456** |
| **250** | **RPL-D1-000177-B04** | **5.2** | **5** | **0.25** | **154.022018** |
| **3362** | **RPL-D1-000216-B03** | **5.2** | **5** | **0.25** | **153.229059** |

### [Example 2] Structural analysis of compounds and alignment based on drug likeness criteria (in silico analysis)

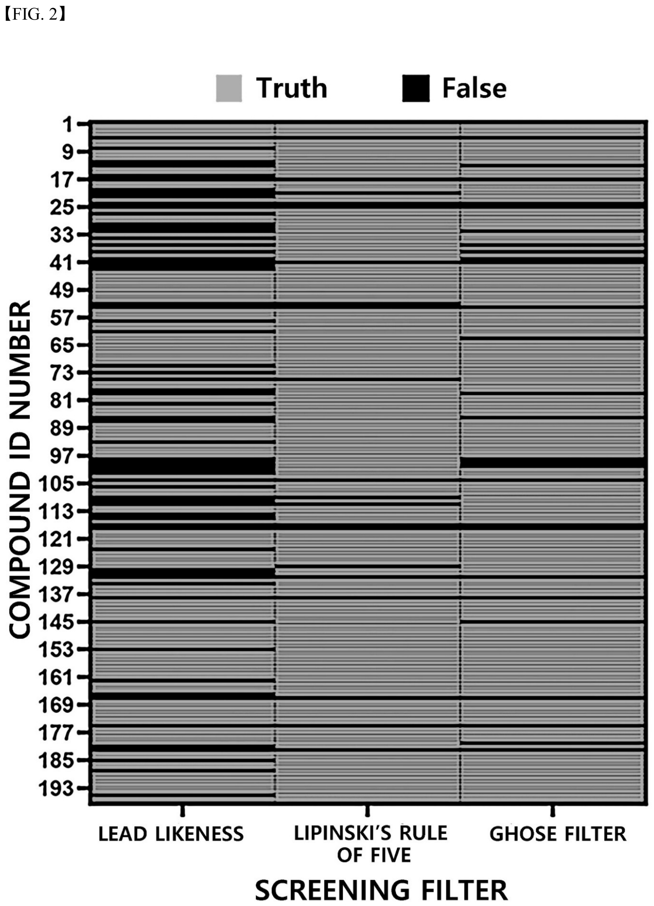

Drug likeness was calculated using ChemoAxon (Table 2), and compounds satisfying lead likeness, Lipinski rule, and Ghose filter were selected (FIG. 2). The proportion of candidate compounds that showed 3, 2, 1, and 0 violations of the three above-mentioned virtual screening filter criteria was 7.1%, 8.1%, 19.8%, and 65.0%, respectively. Structural analysis of the candidate compounds showed that 1) they were compounds with high hydrophobicity that could easily penetrate lipid membranes or remain in the membrane while operating and 2) they had aromatic groups such as benzene rings, suggesting a potential role as a radical scavenger. Thirty compounds were likely not radical scavengers and likely had the possibility of specific binding. A total of 46 compounds were selected, including 34 compounds that were thought to be radical scavengers, among those that satisfied all drug likeness criteria.

**[Table 2]**

| **Drug likeness criteria** | | | |
|---|---|---|---|
| Property | Lead likeness | Lipinski's Rule of Five (4 of 4) | Ghose Filter |
| MW | <= 450 | <= 500 | 160 <= x <= 480 |
| HB donor count | <= 5 | <= 5 | |
| HB acceptor count | <= 8 | <= 10 | |
| LogD (pH 7.4) | -4 <= x <= 4 | | |
| Ring count | <= 4 | | |
| Rotatable bond count | <= 10 | | |
| LogP | | <= 5 | - 0.4 <= x <= 5.6 |
| Refractivity | | | 40 <= x <= 130 |
| Atom count | | | 20 <= x <= 70 |
| References: | Teague, S. L. et al., Angew. Chem. Int. Ed. 1999, 38, 3743. | Adv. Drug Deliv. Rev., 23, 3 (1997). | J. Comb. Chem., 1, 55 (1999). |

### [Example 3] Selection of final compound using real-time live-cell imaging technology

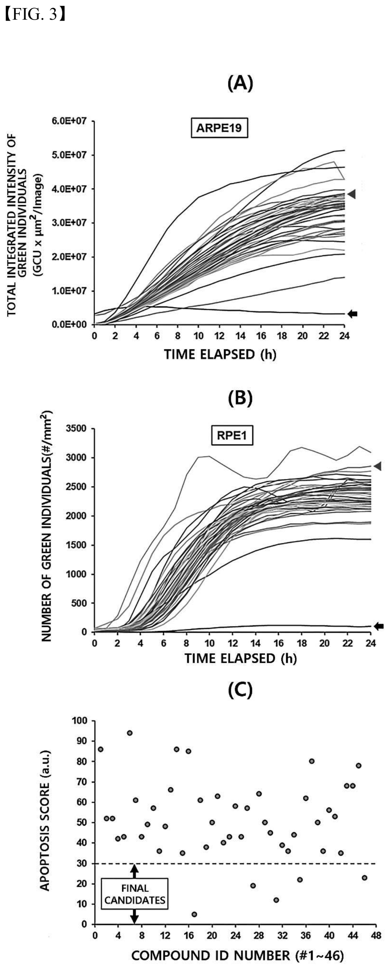

To determine the real-time cytoprotective effects of the final candidate compounds against all-trans-retinal cytotoxicity, ARPE-19 and RPE-1 cells were treated with all-trans-retinal at a concentration of 30 µM. The cells were then treated with the 46 candidate compounds selected in Example 2. Real-time cell viability was measured using Incucyte for 24 hours (FIGS. 3A and 3B). The viability ranks for each cell line were combined and designated an apoptosis score. The five final candidate compounds were then sorted in ascending order of apoptosis scores (i.e., descending order of cell viability) to select the final five candidates (FIG. 3C).

### [Example 4] Confirmation of structure and cytotoxicity of five final candidate compounds

The structures of the five final candidate compounds selected through real-time live cell imaging technology performed in Example 3 are show below.

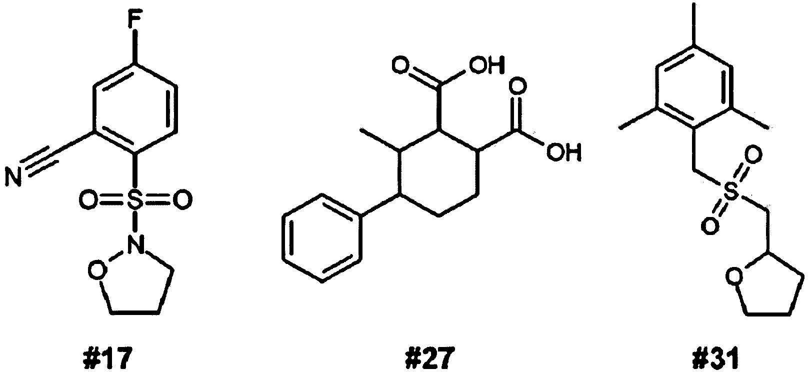

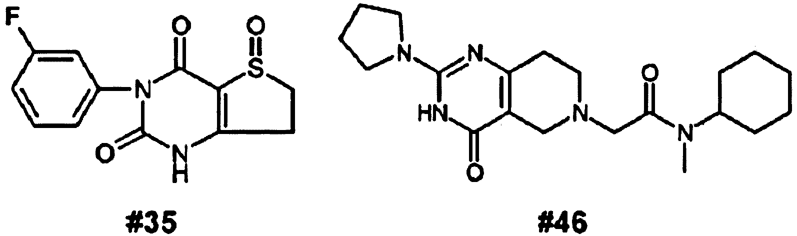

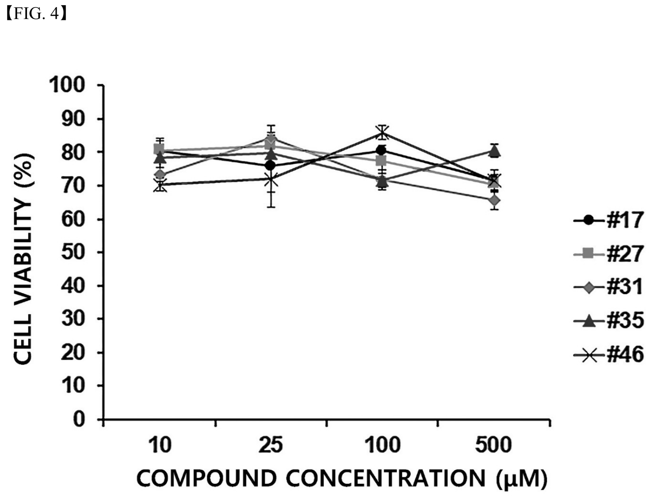

To confirm the cytotoxicity of the final candidate compounds themselves, RPE-1 cells were treated with the candidate compounds at various concentrations (10, 25, 100, 500 µM) and cultured for 24 hours. As a result, relatively good cell viability was confirmed even at high concentrations of the compounds (FIG. 4), confirming that the candidate compounds themselves had minimal toxicity. Compared to the control group, there was no difference in the amount of apoptosis-related factors (such as caspase-9) when treated with candidate compounds, and there were no particular morphological changes in the cells. Bulk RNA-sequencing did not show any significant inflammation or increased expression of apoptosis-related RNAs.

### [Example 5] Cellular aging analysis with five final candidate drugs

It is known that premature senescence occurs when retinal pigment epithelial cells are treated with low, sub-cytotoxic concentrations of hydrogen peroxide (H₂O₂). Using this, 100 µM H₂O₂ was applied to ARPE-19 cells, which were then treated with the candidate compounds to determine their senescence inhibition effects.

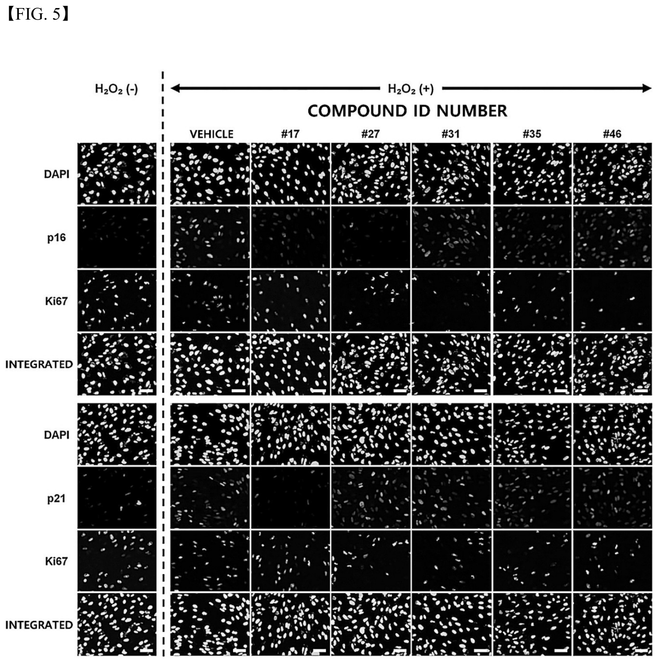

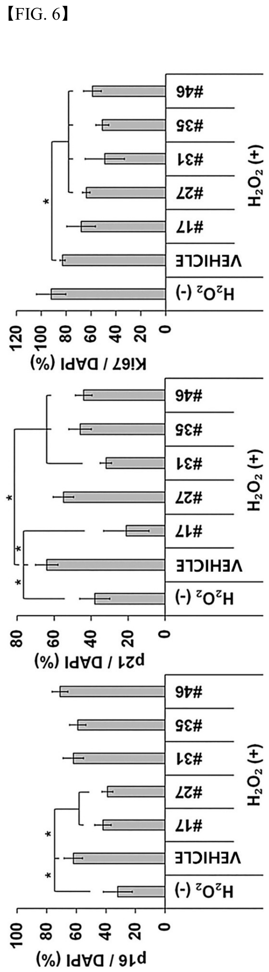

As shown in FIGS. 5 and 6, the expression of p16 and p21, representative senescence markers, was increased in ARPE-19 cells after hydrogen peroxide treatment. A decrease in p16 was observed with compounds #17 and #27, while a decrease in p21 was observed with compounds #17, #31, #35, and #46.

The representative proliferation marker, ki67, showed a decrease in expression in all candidate compound treatment groups. However, the decrease was less pronounced in compound #17, and there was no statistically significant difference compared to the group treated with a negative control substance (vehicle).

Considering these results, compound #17 was determined to have the strongest cytoprotective and senescence inhibition effects, and further experiments were conducted on compound #17.

### [Example 6] Confirmation of reactive oxygen species inhibitory effect of compound #17

When all-trans-retinal was applied to RPE-1 cells, an increase in intracellular reactive oxygen species was observed. To confirm the intracellular reactive oxygen species inhibitory effect of compound #17, a DCFDA assay was performed.

When compound #17 was added to cells, it effectively inhibited the increase in intracellular reactive oxygen species induced by all-trans-retinal, reducing intracellular reactive oxygen species levels to levels almost below those of normal cells without all-trans-retinal.

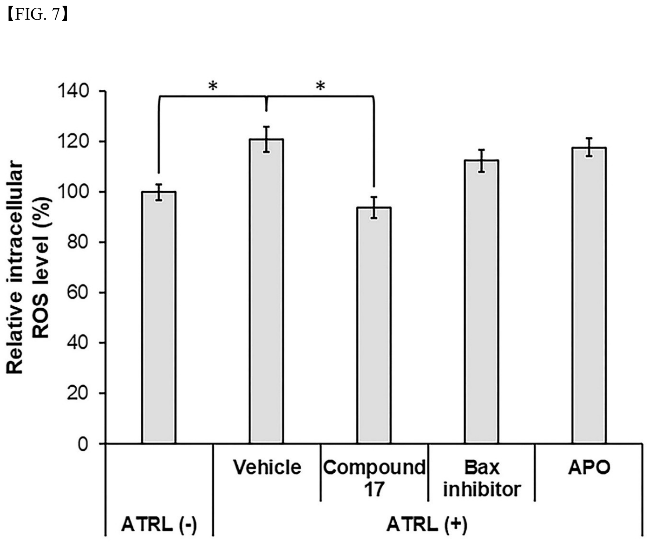

Compared to Bax inhibitor, an anti-apoptotic protein that suppresses intracellular reactive oxygen species accumulation, and apocynin (APO), an antioxidant that inhibits NADPH oxidase, it was confirmed that compound #17 exhibited significantly superior reactive oxygen species inhibition activity (FIG. 7).

## Claims

1. A composition for preventing, ameliorating, or treating retinal diseases, comprising as an active ingredient one or more compounds represented by the following General Formulas 1 to 5: 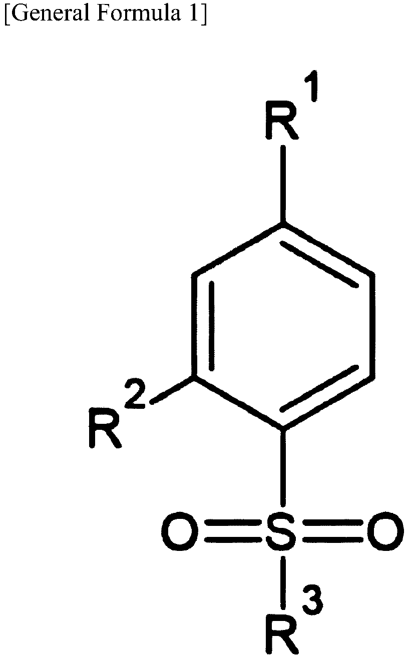 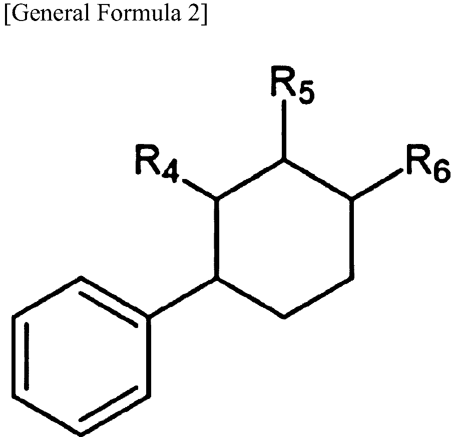 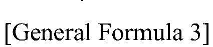 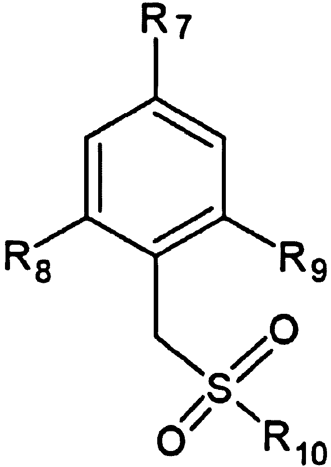 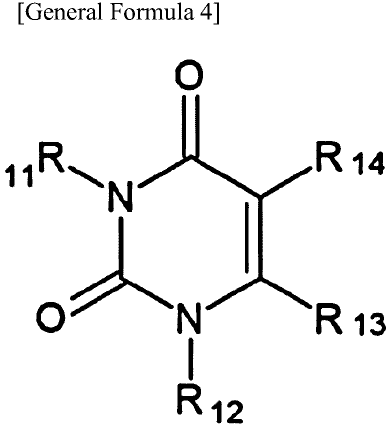 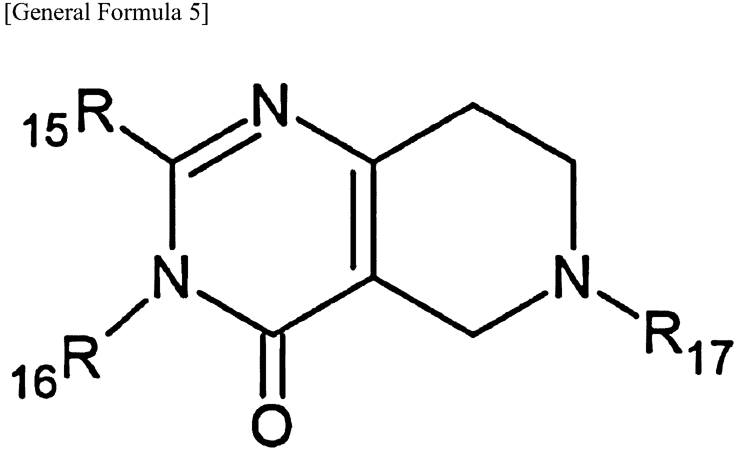 wherein R1 to R17 of General Formulas 1 to 5 are each a substituent selected from the group consisting of a hydrogen, a deuterium, a halogen atom, a hydroxyl group, a cyano group, an amino group, an amido group, a nitro group, a substituted or unsubstituted (C1-C30) alkyl group, a substituted or unsubstituted (C3-C30) cycloalkyl group, a substituted or unsubstituted (C1-C30) heteroalkyl group, a substituted or unsubstituted (C2-C30) heterocycloalkyl group, a substituted or unsubstituted (C6-C30) aryl (C1-C30) alkyl group, an alkoxy group, an aryloxy group, a silyl group, a substituted or unsubstituted (C2-C30) alkenyl group, a substituted or unsubstituted (C2-C30) cycloalkenyl group, a substituted or unsubstituted (C1-C30) heteroalkenyl group, a substituted or unsubstituted (C2-C30) alkynyl group, a substituted or unsubstituted (C6-C30) aryl group, a substituted or unsubstituted (C2-C30) heteroaryl group, an acyl group, a carboxylic acid, an ether, an ester, a nitrile, an isonitrile, a sulfanyl group, a sulfinyl group, a sulfonyl group, a phosphino group, and combinations thereof.

2. The composition of claim 1, wherein R1 of General Formula 1 is a substituent selected from the group consisting of a hydrogen, a deuterium, a halogen atom, and combinations thereof,
R2 is a substituent selected from the group consisting of a hydroxyl group, a cyano group, an amino group, an amido group, a nitro group, an alkoxy group, an aryloxy group, a silyl group, an acyl group, a carboxylic acid group, an ether group, an ester group, a nitrile, an isonitrile, a sulfanyl group, a sulfinyl group, a sulfonyl group, a phosphino group, and combinations thereof, and
R3 is a substituent selected from the group consisting of a substituted or unsubstituted (C1-C30) heteroalkyl group, a substituted or unsubstituted (C2-C30) heterocycloalkyl group, a substituted or unsubstituted (C1-C30) heteroalkenyl group, a substituted or unsubstituted (C2-C30) heteroaryl group, and combinations thereof.

3. The composition of claim 1, wherein R4 of General Formula 2 is a substituent selected from the group consisting of a hydrogen, a deuterium, a substituted or unsubstituted (C1-C30) alkyl group, and combinations thereof, and
R5 or R6 is each a substituent selected from the group consisting of a hydroxyl group, a cyano group, an amino group, an amido group, a nitro group, an alkoxy group, an aryloxy group, a silyl group, an acyl group, a carboxylic acid, an ether, an ester, a nitrile, an isonitrile, a sulfanyl group, a sulfinyl group, a sulfonyl group, a phosphino group, and combinations thereof.

4. The composition of claim 1, wherein R7 to R9 of General Formula 3 are each a substituent selected from the group consisting of a hydrogen, a deuterium, a substituted or unsubstituted (C1-C30) alkyl group, and combinations thereof, and
R10 is a substituent selected from the group consisting of a substituted or unsubstituted (C1-C30) alkyl group, a substituted or unsubstituted (C3-C30) cycloalkyl group, a substituted or unsubstituted (C1-C30) heteroalkyl group, a substituted or unsubstituted (C2-C30) heterocycloalkyl group, a substituted or unsubstituted (C6-C30) aryl (C1-C30) alkyl group, a substituted or unsubstituted (C1-C30) heteroalkenyl group, a substituted or unsubstituted (C2-C30) heteroaryl group, and combinations thereof.

5. The composition of claim 1, wherein R11 of General Formula 4 is a substituent selected from the group consisting of a substituted or unsubstituted (C1-C30) alkyl group, a substituted or unsubstituted (C3-C30) cycloalkyl group, a substituted or unsubstituted (C1-C30) heteroalkyl group, a substituted or unsubstituted (C2-C30) heterocycloalkyl group, a substituted or unsubstituted (C6-C30) aryl (C1-C30) alkyl group, a substituted or unsubstituted (C2-C30) alkenyl group, a substituted or unsubstituted (C2-C30) cycloalkenyl group, a substituted or unsubstituted (C1-C30) heteroalkenyl group, a substituted or unsubstituted (C2-C30) alkynyl group, a substituted or unsubstituted (C6-C30) aryl group, a substituted or unsubstituted (C2-C30) heteroaryl group, and combinations thereof,
R12 is a substituent selected from the group consisting of a hydrogen, a deuterium, a substituted or unsubstituted (C1-C30) alkyl group, and combinations thereof, and
R13 and R14 are fused to form a substituted or unsubstituted ring.

6. The composition of claim 1, wherein R15 of General Formula 5 is a substituent selected from the group consisting of a substituted or unsubstituted (C1-C30) alkyl group, a substituted or unsubstituted (C3-C30) cycloalkyl group, a substituted or unsubstituted (C1-C30) heteroalkyl group, a substituted or unsubstituted (C2-C30) heterocycloalkyl group, a substituted or unsubstituted (C6-C30) aryl (C1-C30) alkyl group, a substituted or unsubstituted (C1-C30) heteroalkenyl group, a substituted or unsubstituted (C2-C30) heteroaryl group, and combinations thereof,
R16 is a substituent selected from the group consisting of a hydrogen, a deuterium, a substituted or unsubstituted (C1-C30) alkyl group, and combinations thereof, and
R17 is a substituent selected from the group consisting of a substituted or unsubstituted (C1-C30) alkyl group, a substituted or unsubstituted (C3-C30) cycloalkyl group, a (C1-C30) heteroalkyl group, a substituted or unsubstituted (C2-C30) heterocycloalkyl group, an amino group, an amido group, a nitro group, a carboxylic acid, an ester, and combinations thereof.

7. The composition of claim 1, wherein the retinal disease is one or more selected from the group consisting of hereditary macular degeneration, age-related macular degeneration, myopic macular degeneration, and macular degeneration caused by inflammation.

8. The composition of claim 7, wherein the hereditary macular degeneration is one or more selected from the group consisting of retinitis pigmentosa, vitelliform macular dystrophy, Stargardt disease, X-linked retinoschisis, and cone dystrophy.

9. The composition of claim 7, wherein the macular degeneration caused by inflammation is macular degeneration caused by Behcet's uveitis

10. The composition of claim 1, wherein the composition includes the compound at a concentration of 0.1 µM to 1000 µM.

11. The composition of claim 1, wherein the compound has one or more effects of cell death inhibition, cell aging inhibition, or intracellular reactive oxygen species inhibition.

12. The composition of any one of claims 1 to 11, wherein the composition is one or more of a pharmaceutical composition, a quasi-drug composition, and a health functional food composition.
